(19) Europäisches Patentamt
European Patent Office
Office européen des brevets

(11) Publication number: **0 612 737 A1**

(12) **EUROPEAN PATENT APPLICATION**

(21) Application number: **94200974.7**

(51) Int. Cl.[5]: **C07D 281/10**, //A61K31/55

(22) Date of filing: **10.04.90**

This application was filed on 11 - 04 - 1994 as a divisional application to the application mentioned under INID code 60.

(30) Priority: **28.04.89 JP 109794/89**

(43) Date of publication of application: **31.08.94 Bulletin 94/35**

(60) Publication number of the earlier application in accordance with Art.76 EPC: **0 395 302**

(84) Designated Contracting States: **AT BE CH DE DK ES GB GR IT LI LU NL SE**

(71) Applicant: **TANABE SEIYAKU CO., LTD.**
**2-10, Dosho-machi 3-chome**
**Chuo-ku Osaka (JP)**

(72) Inventor: **Nishimoto, Shigeru**
**B-110, 16 Aogein 1-chome**
**Minoo-shi, Osaka-fu (JP)**
Inventor: **Nakao, Akio**
**15-4 Abeno-motomachi,**
**Abeno-ku**
**Osaka-shi, Osaka (JP)**
Inventor: **Ikeda, Yasuji**
**501, 7-8 Higashino-Kitsuneyabu-cho,**
**Yamashina-ku**
**Kyoto-shi, Kyoto-fu (JP)**
Inventor: **Nate, Hiroyuki**
**304, 9-3 Kosaka-Honmachi 2-chome**
**Higashiosaka-shi, Osaka-fu (JP)**

(74) Representative: **Hardisty, David Robert et al**
**BOULT, WADE & TENNANT**
**27 Furnival Street**
**London EC4A IPO (GB)**

(54) **Process for preparing 1,5-benzothiazepine derivatives.**

(57) There is disclosed a process for preparing 1,5-benzothiazepine derivatives represented by the formula:

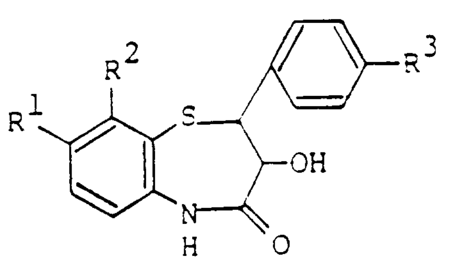

(I)

wherein one of $R^1$ and $R^2$ is a lower alkyl group or halogen atom, and the other is hydrogen atom, $R^3$ is a lower alkyl group or a lower alkoxy group,
which comprises subjecting a propionic acid compound represented by the formula (II):

EP 0 612 737 A1

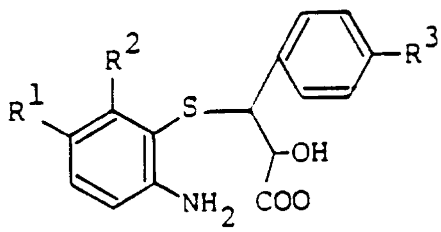

(II)

wherein $R^1$, $R^2$ and $R^3$ have the same meanings as defined above,
to intramolecular ring closing reaction in the presence of a sulfonic acid compound represented by the formula (III):

$R^5SO_3H$ (III)

wherein $R^5$ represents a lower alkyl group or a substituted or unsubstituted phenyl group.

This invention relates to a novel process for preparing 1,5-benzothiazepine derivatives represented by the formula:

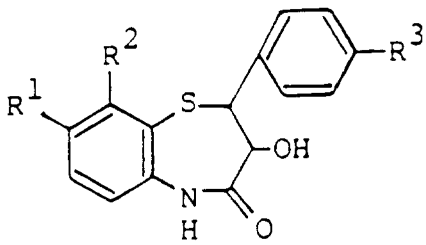

(I)

wherein one of $R^1$ and $R^2$ is a lower alkyl group or halogen atom, and the other is hydrogen, $R^3$ is a lower alkyl group or a lower alkoxy group.

The above 1,5-benzothiazepine derivatives (I) are useful as an intermediate for the synthesis of, for example, the corresponding 3-acetoxy-5-[2-(dimethylamino)ethyl]-1,5-benzothiazepine derivatives having excellent hypotensive activity.

Heretofore, as a process for preparing 1,5-benzothiazepine derivatives (I), the method in which a propionic acid derivatives represented by the formula:

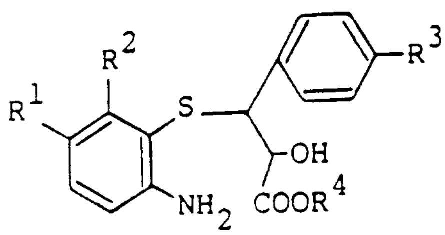

(II)

wherein $R^4$ represents hydrogen atom or an ester residue, and $R^1$, $R^2$ and $R^3$ have the same meanings as defined above,
is heated in a solvent (for example, xylene) to effect intramolecular ring closing has been known (U.S. Patents No. 4,567,175 and No. 4,590,188). However, this method involves the problem of requiring a long period of time for the intramolecular ring closing reaction.

The present inventors have studied various research and as a result, they have found that, when the intramolecular ring closing reaction of the compound (II) is carried out in the presence of a specific sulfonic acid compound, the compound (I) can be prepared by the reaction within a short time with good yield. The present invention has been established based on such findings.

That is, according to the process of the present invention, the 1,5-benzothiazepine derivatives represented by the formula (I) can be prepared by subjecting a propionic acid derivative represented by the formula (II):

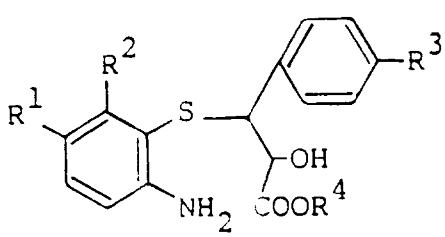

(II)

wherein $R^1$, $R^2$, $R^3$ and $R^4$ have the same meanings as defined above,
to intramolecular ring closing reaction in the presence of a sulfonic acid compound represented by the formula (III):

$R^5SO_3H$ (III)

wherein $R^5$ represents a lower alkyl group or a substituted or unsubstituted phenyl group.

Examples of the sulfonic acid compound (III) to be used in the intramolecular ring closing reaction of the present invention include, for example, the compounds wherein $R^5$ in the formula (III) is an alkyl group having 1 to 4 carbon atoms such as methyl group, ethyl group, propyl group or butyl group or a phenyl group which may be substituted by at least one of these alkyl groups, and particularly, methanesulfonic acid or p-toluenesulfonic acid is preferably used. An amount of the said sulfonic acid is not particularly limited but generally it is preferably used at an amount of 0.5 to 10 w/w %, more preferably about 1 to about 6 w/w % based on the compound (II).

The present intramolecular ring closing reaction is preferably practiced in an appropriate solvent under reflux. As the solvent, high boiling point solvents such as xylene, toluene and dichlorobenzene are preferably used, and xylene is particularly preferably used. A reaction time may be extremely short as compared with the case where no sulfonic acid compound is present in the reaction system, and for example, when xylene is used as a solvent, the reaction can be terminated at about 30 minutes to about 4 hours.

The desired compound (I) formed can be isolated as a pure product containing no sulfonic acid compound (III) by simple and easy operations as, for example, cooling the reaction mixture, collecting precipitated crystals by filtration, and washing with a suitable solvent (e.g. ethanol, aqueous ethanol, etc.).

The thus obtained compound (I) can be converted to the corresponding 3-acetoxy-5-(β-dimethylaminoethyl)-1,5-benzothiazepine derivatives represented by the formula:

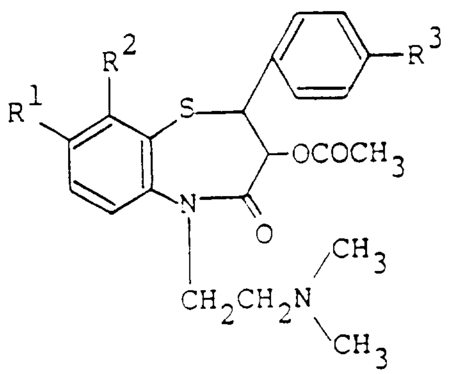

(VI)

wherein $R^1$, $R^2$ and $R^3$ have the same meanings as defined above,
or a pharmaceutically acceptable salt thereof in a known method, for example, in a method described in U.S. Patents No. 4,567,175 and No. 4,590,188, the contents of which are hereby incorporated herein by reference.

According to the process of the present invention as mentioned above, the intramolecular ring closing reaction can be terminated within a short period of time and the desired compound can be obtained in high yield and high purity. Therefore, the process of the present invention is extremely excellent from the industrial view point as compared with the conventional process in which the reaction is performed in the absence of the sulfonic acid compound.

The starting compound (II) can be prepared according to the method as disclosed in Japanese Provisional Patent Publication No. 225174/1984 or No. 202871/1985, but an optically active compound (II) wherein $R^4$ is an ester residue, i.e. an optically active threo-propionic acid ester derivative represented by the formula:

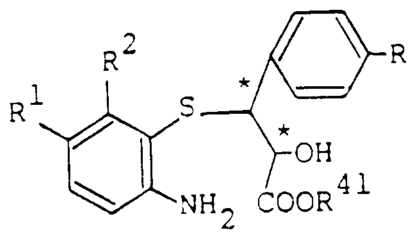

(II - a)

wherein $R^{41}$ represents an ester residue, * represents said carbon atom is an asymmetric carbon atom, and $R^1$, $R^2$ and $R^3$ have the same meanings as defined above,
can be prepared by reacting a thiophenol compound represented by the formula:

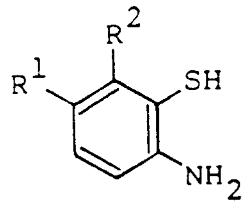

(IV)

wherein symbols have the same meanings as defined above,
with an optically active trans-glycidic acid ester represented by the formula:

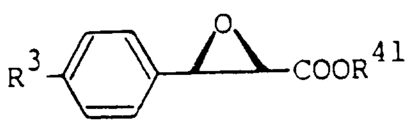

(V)

wherein symbols have the same meanings as defined above.

The above reaction is preferably practiced in an appropriate solvent (e.g. xylene, toluene, etc.) under heating. The compound (II - a) may be isolated, but without isolation, the reaction mixture can be applied to the subsequent intramolecular ring closure reaction. In this case, the optically active desired compound (I) can be obtained from the compound (IV) in the same reaction vessel within a short period of time in good yield. Therefore, the process is industrially extremely advantageous.

Throughout the specification and claims, the term "lower alkyl" and "lower alkoxy" should be interpreted as referring to alkyl having one to four carbon atoms and alkoxy having one to four carbon atoms, respectively.

Example 1

(1) In 400 ml of toluene are dissolved 24.0 g of 2-amino-5-chlorothiophenol and 31.2 g of (-)-trans-3-(4-methoxyphenyl)glycidic acid methyl ester and the solution is refluxed under nitrogen atmosphere for 2 hours. Diisopropyl ether is added to the reaction mixture, and precipitated crystals are collected by filtration to give 37.6 g of (+)-threo-2-hydroxy-3-(2-amino-5-chlorophenylthio)-3-(4-methoxyphenyl)-propionic acid methyl ester as colorless needles.

Yield: 68 %
m.p. 126 to 129.5 °C
$[\alpha]_D^{20}$ + 248.8 ° (c = 0.3, methanol)

(2) A mixture of 9.5 g of the present product, 0.49 g of p-toluenesulfonic acid monohydrate and 95 ml of xylene is refluxed for 2 hours. After cooling, precipitated crystals are collected by filtration to give 7.5 g of (+)-cis-2-(4-methoxyphenyl)-3-hydroxy-8-chloro-2,3-dihydro-1,5-benzothiazepin-4(5H)-one.

Yield: 86.8 %
m.p. 244 to 245 °C
$[\alpha]_D^{20}$ + 91.6 ° (c = 1.0, dimethylformamide)

Example 2

A mixture of 17.0 g of (+)-threo-2-hydroxy-3-(2-amino-5-chlorophenylthio)-3-(4-methoxyphenyl)-propionic acid, 0.91 g of p-toluenesulfonic acid monohydrate and 220 ml of xylene is refluxed for 2 hours under continuous removal of water. After cooling, precipitated crystals are collected by filtration and washed with ethanol to give 15 g of (+)-cis-2-(4-methoxyphenyl)-3-hydroxy-8-chloro-2,3-dihydro-1,5-benzothiazepin-4(5H)-one.

Yield: 93 %

m.p. 244 to 245 °C

Incidentally, according to the method in which the above reaction is practiced in the absence of p-toluenesulfonic acid in the same manner as described in Japanese Provisional Patent Publication No. 225174/1984, yield of the desired compound is 73 % even after refluxing for 20 hours.

Example 3

A mixture of 17.0 g of (+)-threo-2-hydroxy-3-(2-amino-5-chlorophenylthio)-3-(4-methoxyphenyl)-propionic acid, 0.91 g of p-toluenesulfonic acid monohydrate and 220 ml of toluene is refluxed for 8 hours under continuous removal of water. After cooling, precipitated crystals are collected by filtration and washed with ethanol to give 14.8 g of (+)-cis-2-(4-methoxyphenyl)-3-hydroxy-8-chloro-2,3-dihydro-1,5-benzothiazepin-4(5H)-one.

Yield: 92 %

m.p. 244 to 245 °C

Example 4

(1) In 600 ml of xylene are dissolved 132 g of 2-amino-5-methylthiophenol and 200 g of (±)-trans-3-(4-methylphenyl)glycidic acid methyl ester and the solution is refluxed at 120 to 130 °C under nitrogen atmosphere for 4 hours. After cooling to 40 °C, n-hexane is added to the reaction mixture, and the reaction mixture is stirred and then cooled to 10 °C. Precipitated crystals are collected by filtration to give 218 g of (±)-threo-2-hydroxy-3-(2-amino-5-methylphenylthio)-3-(4-methylphenyl)propionic acid methyl ester as colorless crystals.

Yield: 69 %

m.p. 114 to 116 °C

(2) In 500 g of xylene are dissolved 50 g of the product, 0.57 g of p-toluenesulfonic acid monohydrate is added to the solution and the solution is refluxed for 4 hours. After cooling, precipitated crystals are collected by filtration, washed with xylene to give 39.0 g of (±)-cis-2-(4-methylphenyl)-3-hydroxy-8-methyl-2,3-dihydro-1,5-benzothiazepin-4(5H)-one as colorless crystals.

Yield: 86 %

m.p. 185 to 186 °C

Example 5

In a solution of 200 ml of water and 100 g of methanol are suspended 40 g of (±)-threo-2-hydroxy-3-(2-amino-5-methylphenylthio)-3-(4-methylphenyl)propionic acid methyl ester, 7.4 g of potassium hydroxide are added to the suspension and the mixture is stirred for 30 minutes at 50 to 55 °C. Then, 13.8 g of 35 % hydrochloric acid are added dropwise, and 200 ml of water are added thereto, and the mixture is cooled to 10 °C. Precipitated crystals are collected by filtration and washed with water to give 80 g of (±)-threo-2-hydroxy-3-(2-amino-5-methylphenylthio)-3-(4-methyl phenyl)propionic acid (wet material).

This product (80 g) is suspended in 400 ml of toluene, and 0.4 g of p-toluenesulfonic acid monohydrate is added thereto and the mixture is refluxed for 5 hours under continuous removal of water. After cooling, precipitated crystals are collected by filtration, washed with toluene and dried to give 32.5 g of (±)-cis-2-(4-methylphenyl)-3-hydroxy-8-methyl-2,3-dihydro-1,5-benzothiazepin-4(5H)-one.

Yield: 90 %

m.p. 185 to 186 °C

Example 6

In 400 ml of toluene are dissolved 24.0 g of 2-amino-5-chlorothiophenol and 31.2 g of (-)-trans-3-(4-methoxyphenyl)glycidic acid methyl ester, and the solution is refluxed under nitrogen atmosphere for 2 hours. To the reaction mixture are added 1.43 g of p-toluenesulfonic acid hydrate and 350 ml of xylene and the mixture is refluxed for 2 hours and simultaneously 350 ml of the solvent is distillated. After cooling, precipitated crystals are collected by filtration to give 19.8 g of (+)-cis-2-(4-methoxyphenyl)-3-hydroxy-8-chloro-2,3-dihydro-1,5-benzothiazepin-4(5H)-one.

Physical and chemical properties of the product were identical to those of the product obtained in Example 1.

Example 7

(1) In 420 ml of xylene are dissolved 42 g of 2-amino-5-methylthiophenol and 58 g of (+)-trans-3-(4-methylphenyl)glycidic acid methyl ester, and the solution is refluxed under nitrogen atmosphere for 2 hours. After cooling, n-hexane is added to the reaction mixture and precipitated crystals are collected by filtration to give 65 g of (-)-threo-2-hydroxy-3-(2-amino-5-methylphenylthio)-3-(4-methylphenyl)-propionic acid methyl ester as colorless needles.

Yield: 65 %

m.p. 107 to 109 °C

$[\alpha]_D^{20}$ - 235.4 ° (c = 1, methanol)

(2) A mixture of 20 g of the above product, 0.4 g of p-toluenesulfonic acid monohydrate and 160 ml of xylene is refluxed for 5 hours. After cooling, precipitated crystals are collected by filtration to give 15.7 g of (-)-cis-2-(4-methylphenyl)-3-hydroxy-8-methyl-2,3-dihydro-1,5-benzothiazepin-4(5H)-one.

Yield: 87 %

m.p. 207 to 212 °C

$[\alpha]_D^{20}$ - 120 ° (c = 0.3, methanol)

**Claims**

**1.** A process for preparing 1,5-benzothiazepine derivatives represented by the formula:

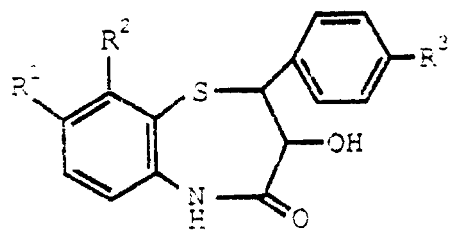

(I)

wherein one of $R^1$ and $R^2$ is a $C_1$-$C_4$ alkyl group or halogen atom, and the other is hydrogen atom, $R^3$ is a $C_1$-$C_4$ alkyl group or a $C_1$-$C_4$ alkoxy group,

which comprises subjecting a propionic acid compound represented by the formula (II):

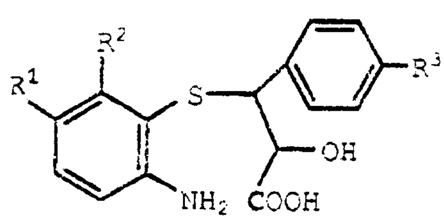

(II)

wherein $R^1$, $R^2$ and $R^3$ have the same meanings as defined above,

to intramolecular ring closing reaction in the presence of a sulfonic acid compound represented by the formula (III):

$R^5SO_3H$ (III)

wherein $R^5$ represents methyl group, ethyl group, propyl group, butyl group or phenyl group which may be substituted by at least one selected from methyl group, ethyl group, propyl group, butyl group or a phenyl group.

**2.** A process for preparing 1,5-benzothiazepine derivatives according to claim 1, wherein the sulfonic acid compound (III) is methanesulfonic acid or p-toluenesulfonic acid.

**3.** A process for preparing 1,5-benzothiazepine derivatives according to claim 1, wherein the reaction is carried out in an appropriate solvent under reflux.

**4.** A process for preparing 1,5-benzothiazepine derivatives according to claim 1, wherein the sulfonic acid (III) is used at an amount of 0.5 to 10 w/w % based on the compound (II).

**5.** A process for preparing 1,5-benzothiazepine derivatives according to claim 4, wherein the sulfonic acid (III) is used at an amount of 1 to 6 w/w % based on the proprionic acid compound (II).

**6.** A process for preparing optically active cis-1,5-benzothiazepine derivatives represented by the formula:

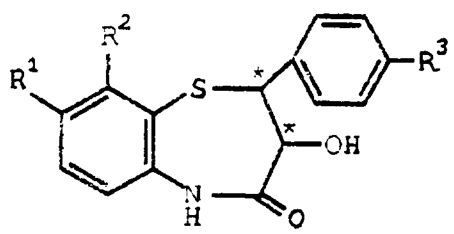

(I)

wherein one of $R^1$ and $R^2$ is a $C_1$-$C_4$ alkyl group or halogen atom, and the other is hydrogen atom, $R^3$ is a $C_1$-$C_4$ alkyl group or a $C_1$-$C_4$ alkoxy group, and * represents said carbon atom in an asymmetric carbon atom,
which comprises reacting a thiophenol compound represented by the formula (IV):

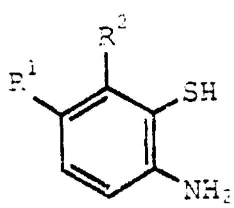

(IV)

wherein $R^1$ and $R^2$ have the same meanings as defined above, with an optically active trans-glycidic acid ester represented by the formula:

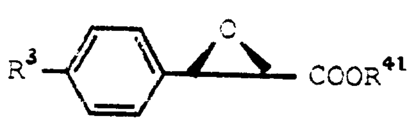

(V)

wherein $R^3$ has the same meaning as defined above, and $R^{41}$ represents an ester residue,
to form an optically active threo-proprionic acid ester compound represented by the formula:

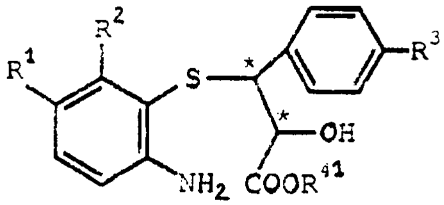

(II - a)

wherein $R^1$, $R^2$, $R^3$, $R^{41}$ and * have the same meanings as defined above, converting the above compound (II-a) to an optically active threo-propionic acid compound represented by the formula:

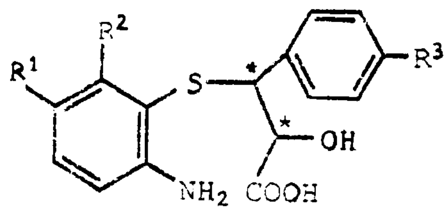

(II - b)

wherein $R^1$, $R^2$, $R^3$, and * have the same meanings as defined above, and then subjecting the above compound (II-b) to intramolecular ring closing reaction in the presence of a sulfonic acid compound represented by the formula (III):

$R^5SO_3H$ (III)

wherein $R^5$ represents methyl group, ethyl group, propyl group, butyl group or phenyl group which may be substituted by at least one selected from methyl group, ethyl group, propyl group and butyl group.

**7.** A process for preparing 1,5-benzothiazepine derivatives according to claim 6, wherein the reaction of the thiophenol compound (IV) and the optically active trans-glycidic acid ester (V) is carried out in an appropriate solvent under heating.

**8.** A process for preparing 1,5-benzothiazepine derivatives according to claim 6, wherein the sulfonic acid compound (III) is methanesulfonic acid or p-toluenesulfonic acid.

**9.** A process for preparing 1,5-benzothiazepine derivatives according to claim 6, wherein the intramolecular ring closing reaction is carried out in an appropriate solvent under reflux.

**10.** A process for preparing 1,5-benzothiazepine derivatives according to claim 6, wherein the sulfonic acid compound (III) is used at an amount of 0.5 to 10 w/w % based on the compound (II-b).

**11.** A process for preparing 1,5-benzothiazepine derivatives according to claim 10, wherein the sulfonic acid compound (III) is used at an amount of 1 to 6 w/w % based on the propionic acid compound (II-b).

**12.** A process for preparing 3-acetoxy-5-(β-dimethylaminoethyl)-1,5-benzothiazepine derivatives represented by the formula:

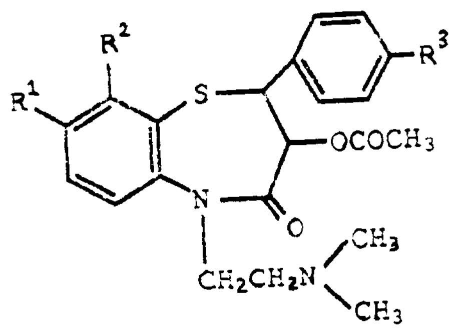

(VI)

wherein one of $R^1$ and $R^2$ is a $C_1$-$C_4$ alkyl group or halogen atom, and the other is hydrogen atom, $R^3$ is a $C_1$-$C_4$ alkyl group or a $C_1$-$C_4$ alkoxy group,
or a pharmaceutically acceptable salt thereof which process comprises subjecting a propionic acid compound represented by the formula (II):

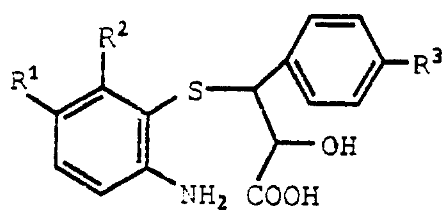

(II)

wherein $R^1$, $R^2$ and $R^3$ have the same meanings as defined above,
to intramolecular ring closing reaction in the presence of a sulfonic acid compound represented by the formula (III):

$R^5SO_3H$ (III)

wherein $R^5$ represents methyl group, ethyl group, propyl group, butyl group or phenyl group which may be substituted by at least one selected from methyl group, ethyl group, propyl group and butyl group,
to give a compound represented by the formula (I):

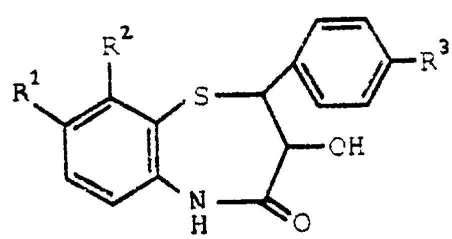

(I)

wherein $R^1$, $R^2$ and $R^3$ have the same meanings as defined above,
and converting the compound (I) to the corresponding 3-acetoxy-5-($\beta$-dimethylaminoethyl)-1,5-benzothiazepine derivative or a pharmaceutically acceptable salt thereof in a known method.

European Patent Office

# EUROPEAN SEARCH REPORT

Application Number
EP 94 20 0974

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| D,A | US-A-4 567 175 (M. TAKEDA ET AL.)<br>* the whole document *<br>--- | 1-12 | C07D281/10<br>//A61K31/55 |
| A | GB-A-2 139 620 (SHIONOGI SEIYAKU KABUSHIKI KAISHA)<br>* the whole document *<br>--- | 1-12 | |
| A | EP-A-0 098 422 (SIEGFRIED AKTIENGESELLSCHAFT)<br>* the whole document *<br>--- | 1 | |
| P,X | EP-A-0 343 474 (F. HOFFMANN-LA ROCHE AG)<br>* the whole document, particularly page 4 , lines 20-35 and page 15, example 14B *<br>----- | 1-12 | |

TECHNICAL FIELDS SEARCHED (Int.Cl.5)

C07D

The present search report has been drawn up for all claims

| Place of search | Date of completion of the search | Examiner |
|---|---|---|
| THE HAGUE | 22 June 1994 | Allard, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)